# EUROPEAN PATENT APPLICATION

(11) **EP 4 597 107 A1**
(43) Date of publication of application: **06.08.2025**
(21) Application number: 23871930.6
(22) Date of filing: 13.09.2023
(51) Int. Cl.: G01N 33/543, G01N 21/76, G01N 21/78, G01N 33/483

(54) **IMMUNOCHROMATOGRAPHY TESTING DEVICE**

(30) Priority: 26.09.2022 JP 2022153101
(71) Applicant: FUJIFILM Corporation, Tokyo 106-8620 (JP)
(72) Inventor: USHIKURA, Shinichi, Ashigarakami-gun, Kanagawa 258-8538 (JP)
(74) Representative: Dehns Germany Partnerschaft mbB
(86) International application number: PCT/JP2023/033373
(87) International publication number: WO 2024/070697

(57) **Abstract**

An immunochromatographic assay apparatus is an immunochromatographic assay apparatus for assaying a sample, and includes a loading part that is configured to attachably and detachably load a cartridge in which an assay strip having an assay region in which the sample is developed and a test substance contained in the sample is captured, and a container that accommodates a treatment liquid to be supplied to the assay region are accommodated in a case, an operation part that is operated by a user, in which in a state where the cartridge is loaded in the loading part, an operation force of the user is applied to the container from an outside of the case as an external force to supply the treatment liquid to the assay region, and a light detection part that detects light from the assay region to which the sample and the treatment liquid are supplied.

## Description

### BACKGROUND OF THE INVENTION

### 1. Field of the Invention

The present disclosure relates to an immunochromatographic assay apparatus.

### 2. Description of the Related Art

An immunological measuring method is widely used as a method of qualitatively or quantitatively measuring a test substance present in a biological specimen such as urine or blood. Among the above, an immunochromatographic method is highly convenient since the operation is easy and the measurement can be carried out in a short time.

In the immunochromatographic method, an assay strip in which an antibody (capture antibody) that specifically binds to a test substance (for example, an antigen) is immobilized in an examination region is used, and light from the assay region is detected to qualitatively or quantitatively measure the antigen as the test substance.

In such an immunochromatographic method, to avoid a problem that a test substance is not detected and shows a false negative due to low sensitivity even though the test substance is contained, a technique for improving sensitivity has been proposed. As one of the techniques for improving sensitivity, JP2009-139254A discloses a technique for amplifying a signal by attaching silver to a metal colloid label. In addition, JP2015-105858A and JP2018-522221A disclose a chemiluminescence immunochromatographic method in which a luminescent protein is used as a label and light caused by a reaction between a luminescent substrate and the luminescent protein is detected.

In a case where a signal amplification technique using silver amplification, which is disclosed in JP2009-139254A, is used, it is possible to perform a highly sensitive detection. In this silver amplification method, it is necessary to supply two types of amplification liquids, that is, a liquid that catalyzes amplification, such as a solution containing a silver ion reducing agent, and a liquid that performs amplification, such as a solution containing a silver ion, to the assay strip.

JP2012-103150A discloses an analysis apparatus (corresponding to an assay apparatus) that can sequentially supply two types of amplification liquids to an assay strip to perform silver amplification as in JP2009-139254A, and that detects an amplified signal from an assay region to determine whether to be positive or negative.

On the other hand, WO2017/104143A proposes an assay cartridge that can perform an assay by a silver amplification method without using an assay apparatus. The cartridge of WO2017/104143A includes two types of amplification liquids in a case, and can sequentially supply the two types of amplification liquids to an assay strip, and can visually determine whether to be positive or negative.

### SUMMARY OF THE INVENTION

In a case where the cartridge of WO2017/104143A is used, the assay can be performed without using an assay apparatus. However, in a case where the test substance contained in the sample is a trace amount, the detection signal may be small even after the amplification treatment, and it may be difficult to visually determine the result. Therefore, an assay apparatus for detecting a change in a color development state of an assay region is desired for a cartridge of a type encompassing two types of treatment liquids.

In addition, a practical cartridge that can easily perform the chemiluminescence immunochromatographic method as disclosed in JP2015-105858A and JP2018-522221A has not been developed so far. Therefore, the practical application of a cartridge that realizes a chemiluminescence immunochromatographic method and an assay apparatus for detecting light from an assay region of the cartridge is expected.

An object of the present disclosed technology is to provide an immunochromatographic assay apparatus that is simple in configuration and low in cost, the immunochromatographic assay device being for determining whether a sample is positive or negative by detecting light from an assay region of a cartridge that encompasses an assay strip having the assay region and a treatment liquid.

An immunochromatographic assay apparatus of the present disclosure is an immunochromatographic assay apparatus for assaying a sample, and includes a loading part that is configured to attachably and detachably load a cartridge in which an assay strip having an assay region in which the sample is developed and a test substance contained in the sample is captured, and a container that accommodates a treatment liquid to be supplied to the assay region are accommodated in a case, an operation part that is operated by a user, in which in a state where the cartridge is loaded in the loading part, an operation force of the user is applied to the container from an outside of the case as an external force to supply the treatment liquid to the assay region, and a light detection part that detects light from the assay region to which the sample and the treatment liquid are supplied.

The treatment liquid includes a first treatment liquid that is supplied to the assay region where the sample has been developed, and a second treatment liquid that is supplied to the assay region after the first treatment liquid is supplied, the container may include a first container that accommodates the first treatment liquid, and a second container that accommodates the second treatment liquid, and the operation part may be an operation part that supplies the second treatment liquid to the assay region by applying an external force to the second container.

The operation part may include a first operation part that supplies the first treatment liquid by applying an external force to the first container, and a second operation part that supplies the second treatment liquid by applying an external force to the second container.

The notification part that notifies of an assay result of the sample may be provided.

The notification part may include a light emitting element and may display whether to be negative or positive by a lighting state of the light emitting element.

The notification part may notify of whether the sample is negative or positive by voice.

A processor that is configured to determine whether the sample is negative or positive based on the signal acquired from the light detection part may be provided.

The light detection part may include an imaging element.

A battery that supplies electricity to the light detection part may be built in.

The notification part may further notify of a timing at which the operation part is operated.

The light detection part may be a light detection part that detects, as the light from the assay region, light caused by a reaction of a luminescent protein which has been applied to the test substance with a luminescent substrate contained in the treatment liquid.

The light detection part may be a light detection part that detects, as the light caused by the reaction of the luminescent protein with the luminescent substrate, fluorescence generated by transferring energy of light generated by the reaction of the luminescent protein with the luminescent substrate, to a fluorescent substance which has been applied to the test substance.

The cartridge may include a photometric window part that outputs the light from the assay region to the outside, and in a case where the cartridge is loaded into the loading part, the cartridge includes, between the photometric window part and the light detection part, an optical filter that attenuates the light generated by the reaction of the luminescent protein with the luminescent substrate, and that allows the fluorescence to be transmitted.

According to the present disclosure, it is possible to provide an immunochromatographic assay apparatus that is simple and low-cost, the immunochromatographic assay apparatus being for determining whether a sample is positive or negative by detecting light from an assay region of a cartridge that encompasses an assay strip having the assay region and a treatment liquid.

### BRIEF DESCRIPTION OF THE DRAWINGS

Fig. 1 is a perspective view of an assay apparatus.
Fig. 2 is a side cross-sectional view of the assay apparatus in a state where a cartridge is loaded.
Fig. 3 is a perspective view of a cartridge.
Fig. 4 is an exploded perspective view of the cartridge.
Fig. 5 is a side cross-sectional view of the cartridge.
Fig. 6 is a side cross-sectional view showing a state in which a first push-down part and a second push-down part of the cartridge are pushed down.
Fig. 7 is a flowchart showing an assay step using the assay apparatus.
Fig. 8 is an explanatory view of BRET.
Fig. 9 is a view showing an assay step of a chemiluminescence immunochromatographic method using BRET.
Fig. 10 is a view showing an assay step of a chemiluminescence immunochromatographic method using BRET.
Fig. 11 is a side cross-sectional view of an assay apparatus of a design modification example.

### DESCRIPTION OF THE PREFERRED EMBODIMENTS

Hereinafter, specific embodiments of the immunochromatographic assay apparatus according to the present disclosure will be described with reference to the drawings. The constituent elements indicated by the same reference numerals in the drawings mean the same constituent elements.

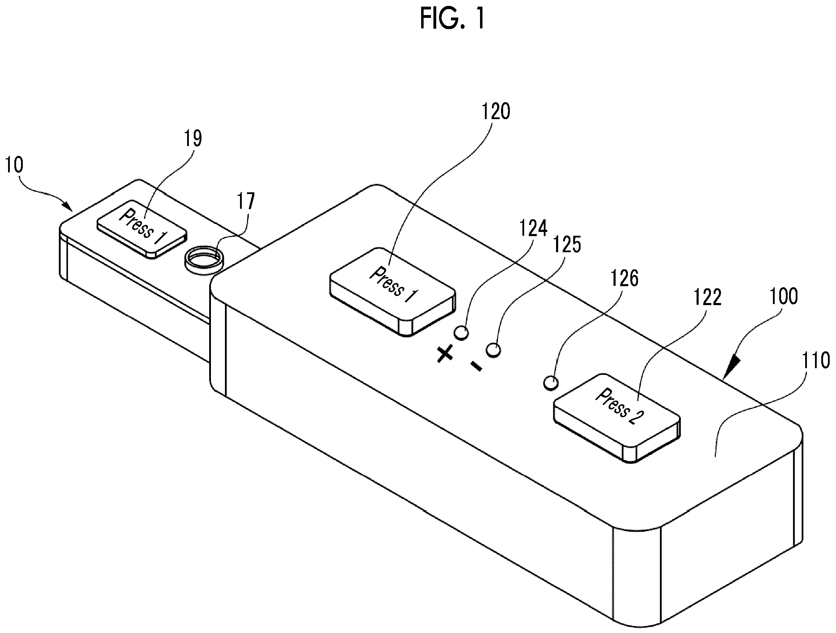

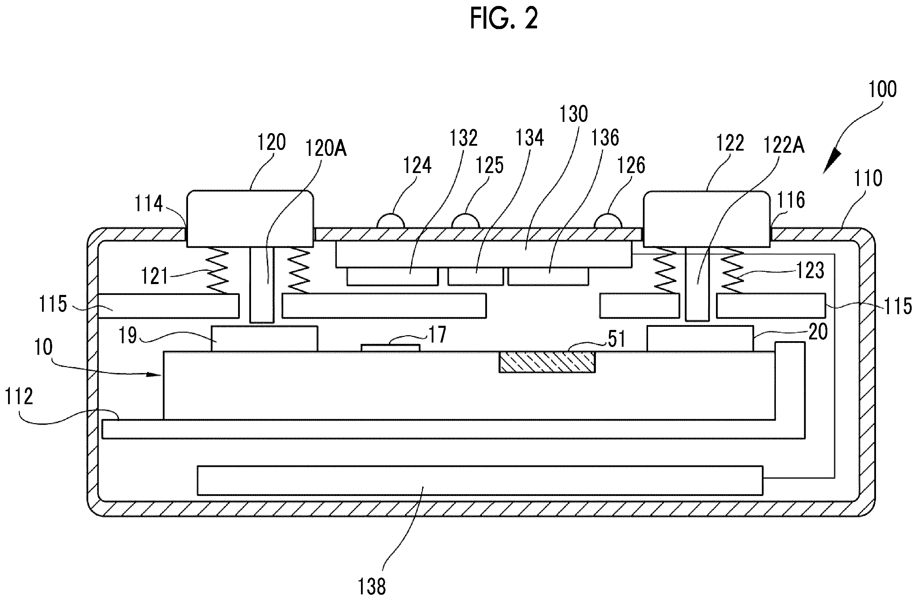

Fig. 1 is a perspective view of an immunochromatographic assay apparatus 100 (hereinafter, simply referred to as an assay apparatus 100) according to one embodiment, and Fig. 2 is a side cross-sectional view of the assay apparatus 100 in which an immunochromatographic cartridge 10 (hereinafter, simply referred to as a cartridge 10) is loaded.

As shown in Fig. 1, the assay apparatus 100 includes a first operation part 120, a second operation part 122, a first light emitting part 124, a second light emitting part 125, and a third light emitting part 126 on one surface (here, an upper surface) of a housing 110. The first operation part 120 is provided on one end side of the housing 110 in the longitudinal direction, and the second operation part 122 is provided on the other end side of the housing 110 in the longitudinal direction. The first light emitting part 124 to the third light emitting part 126 are disposed in order along the longitudinal direction of the housing 110 between the first operation part 120 and the second operation part 122. For example, the first light emitting part 124 is colored red, the second light emitting part 125 is colored green, and the third light emitting part 126 is colored yellow. A symbol "+" is attached to a portion adjacent to the first light emitting part 124 of the upper surface of the housing 110, and a symbol "-" is attached to a portion adjacent to the second light emitting part 125. "+" is a symbol meaning positive, and "-" is a symbol meaning negative. The first light emitting part 124 is turned on in a case of being a positive, and the second light emitting part 125 is turned on in a case of being a negative. The third light emitting part 126 is turned on, for example, in a case where the user is notified of the operation timing of the second operation part 122. It is noted that hereinafter, in a case where the first light emitting part 124, the second light emitting part 125, and the third light emitting part 126 are not distinguished, they may be referred to as the light emitting parts 124 to 126.

As shown in Fig. 2, the assay apparatus 100 includes a loading part 112, a circuit board 130, a processor 132, internal illumination 134, an imaging element 136, and a battery 138 in a housing 110.

The cartridge 10 is attachably and detachably loaded in the loading part 112. A cartridge loading port is provided on one side surface of the housing 110, and the cartridge 10 is loaded and discharged from the one side surface (see Fig. 1).

The assay apparatus 100 determines whether the sample spotted on the cartridge 10 is positive or negative in a state where the cartridge 10 is loaded. Here, the cartridge 10 will be described.

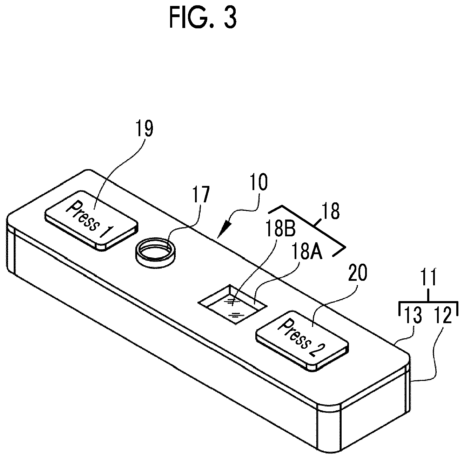

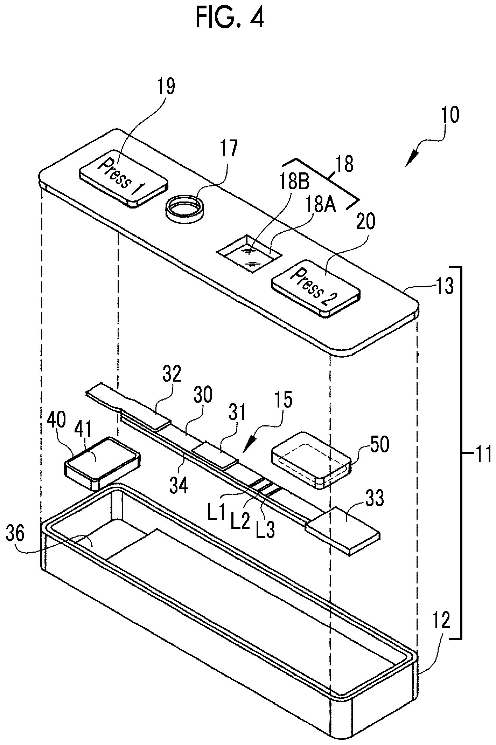

Fig. 3 is a perspective view of the cartridge 10, and Fig. 4 is an exploded perspective view of the cartridge 10. In the cartridge 10, an assay strip 15, a first container 40, and a second container 50 are accommodated in the case 11.

The case 11 is composed of a case main body 12 and a cover member 13. The case main body 12 is a box having an accommodation space that is surrounded by a bottom plate having an elongated rectangular plate shape and four side plates standing vertically from four sides of the bottom plate. An assay strip 15 having the assay region L1 is accommodated in the accommodation space of the case main body 12.

A sample dropping port 17 and a photometric window part 18 are formed in the cover member 13, and a first push-down part 19 and a second push-down part 20 are provided.

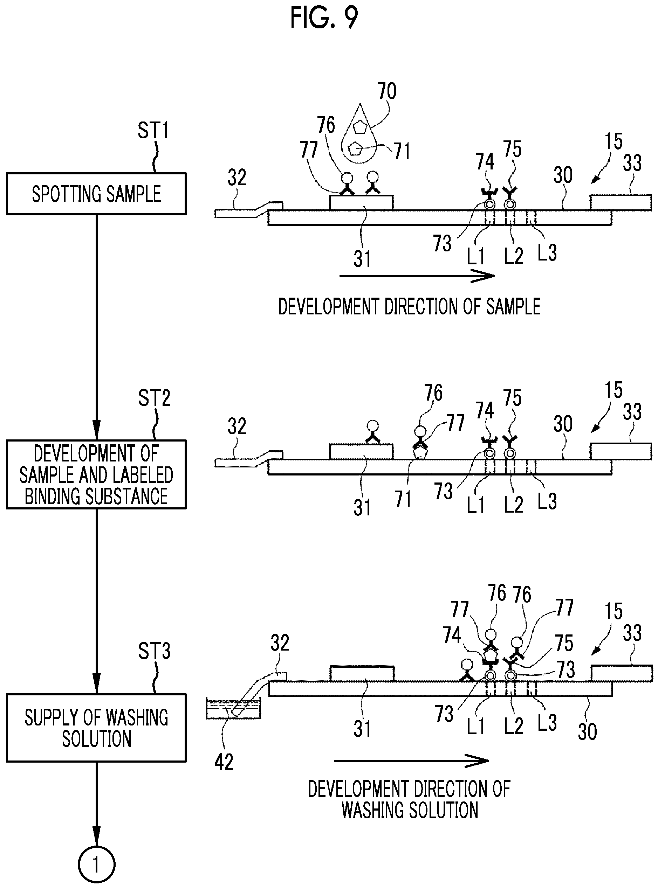

The sample dropping port 17 is a round hole to which a sample solution 70 (see Fig. 9) containing a sample is added dropwise, and has a boss shape in which an edge protrudes from the surface. The sample may be any sample as long as it can contain a test substance 71, and it is not particularly limited. The sample includes a biological specimen of an individual as a target of the immunochromatography assay, particularly an animal as well as a human, for example, blood, serum, blood plasma, spinal fluid, tear fluid, sweat, urine, feces, pus, nasal discharge, nasal swab, pharynx swab, nasal aspirate, or sputum, as well as an organ, a tissue, a mucous membrane, and skin, or swabs containing these, or an animal or plant itself or a dried form thereof. Examples of the test substance 71 include an antigen, an antibody, a protein, and a low-molecular-weight compound.

The photometric window part 18 is a light transmission part for observing the assay region L1 and the like of the assay strip 15 from the outside, and is constituted by a rectangular opening 18A and a transparent member 18B provided at the opening 18A. The photometric window part 18 is formed between the sample dropping port 17 and the second push-down part 20.

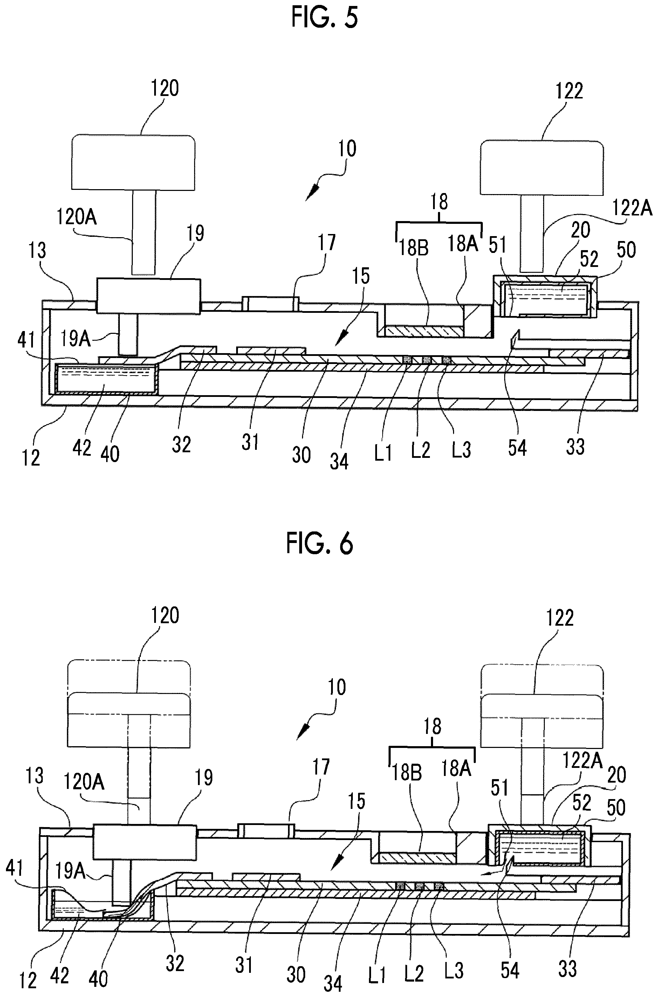

The first push-down part 19 and the second push-down part 20 are provided to be pushable in a direction toward the case main body 12 from a state of protruding from the surface of the cover member 13. The first push-down part 19 is provided at one end part of the cover member 13 in the longitudinal direction. The first push-down part 19 is subjected to push-down operation in a case where the first treatment liquid 42 (see Fig. 5) is supplied to the assay strip 15 (see Figs. 5 and 6). The second push-down part 20 is provided at the other end part of the cover member 13 on a side opposite to the first push-down part 19. The second push-down part 20 is subjected to push-down operation in a case where the second treatment liquid 52 (see Fig. 5) is supplied to the assay strip 15 (see Figs. 5 and 6).

As shown in Fig. 4, the assay strip 15 has an elongated rectangular plate shape as a whole and has a carrier 30, a label holding pad 31, a liquid feeding pad 32, an absorption pad 33, and a back pressure-sensitive adhesive sheet 34.

The carrier 30 is formed from a porous insoluble material such as a nitrocellulose membrane. The label holding pad 31 is attached to the position of the carrier 30 that faces the sample dropping port 17. The sample solution 70 dropped onto the sample dropping port 17 is spotted on the label holding pad 31. That is, the label holding pad 31 functions as a spotting region of the sample solution 70.

The sample solution 70 spotted on the label holding pad 31 permeates the carrier 30 and develops to one end side of the carrier 30 in the longitudinal direction because of a capillary action. As an example, an assay region L1, a control region L2, and a color development region L3 are provided on one end side of the carrier 30 in the longitudinal direction to which the sample solution 70 is developed. The assay region L1, the control region L2, and the color development region L3 are strip-shaped regions extending from one end to the other end of the carrier 30 in the width direction. In a case where a direction from the label holding pad 31 toward the assay region L1 and the like is defined as the development direction of the sample solution 70, the assay region L1 is positioned on the most upstream side in the development direction and the color development region L3 is positioned on the most downstream side in the development direction. The control region L2 is positioned between the assay region L1 and the color development region L3. In other words, the control region L2 is positioned on the downstream side of the assay region L1 and on the upstream side of the color development region L3. In Fig. 4, the assay region L1, the control region L2, and the color development region L3 are hatched, but the hatching is for convenience of description and does not indicate that each of the regions L1 to L3 develops color. The same applies to Fig. 5, Fig. 6, and the like.

A labeled binding substance 77 (see Fig. 9) to which a label is attached is provided in the label holding pad 31. The labeled binding substance 77 is a substance that specifically binds to the test substance 71. Examples of the label include a metal colloid, a fluorescent particle, a luminescent protein, and the like. For example, in a case where the test substance 71 is an antigen, the labeled binding substance 77 is an antibody against the antigen, and in a case where the test substance 71 is an antibody, the labeled binding substance 77 is an antigen against the antibody. In a case where the test substance 71 is a protein, a low-molecular-weight compound, or the like, the labeled binding substance 77 is an aptamer with respect to the protein, the low-molecular-weight compound, or the like.

A binding substance for capturing 74 (see Fig. 9) that specifically binds to the test substance 71 is immobilized in the assay region L1. For example, in a case where the test substance 71 is an antigen, the binding substance for capturing 74 is an antibody against the antigen, and in a case where the test substance 71 is an antibody, the binding substance for capturing 74 is an antigen against the antibody. In a case where the test substance 71 is a protein, a low-molecular-weight compound, or the like, the binding substance for capturing 74 is an aptamer with respect to the protein, the low-molecular-weight compound, or the like. The binding substance for capturing 74 may be the same as or different from the labeled binding substance 77. In a case where the sample includes the test substance 71, the label is captured in the assay region L1 via the test substance 71. It is possible to determine whether the sample is positive or negative by performing a photometry of the light caused by the label from the assay region L1.

A control binding substance 75 (see Fig. 9) that specifically binds to the labeled binding substance 77 is immobilized in the control region L2. The control binding substance 75 may be the test substance 71 itself or may be a compound having a moiety that is recognized by the labeled binding substance 77. The labeled binding substance 77 that has not bound to the test substance 71 is captured in the control region L2. Since the control region L2 captures the labeled binding substance 77 even in a case where the sample does not contain the test substance 71, it is possible to confirm that the sample has reliably reached the assay region L1 and the control region L2 by performing a photometry of the light from the control region L2.

The color development region L3 contains a substance (not shown) of which the color development state changes by reacting with the first treatment liquid 42. In a case where the color development state of the color development region L3 changes by reacting with the first treatment liquid 42, this indicates that the first treatment liquid 42 is developed in the assay region L1 and the control region L2.

The liquid feeding pad 32 is attached to one end of the carrier 30, which is on a side opposite to the other end of the carrier 30 having the assay region L1 or the like. The liquid feeding pad 32 is formed from a porous material similarly to the carrier 30 and the like, and it feeds the first treatment liquid 42 to the carrier 30 by the capillary action.

The absorption pad 33 is attached to one end on a side opposite to the other end of the carrier 30 to which the label holding pad 31 is attached. The absorption pad 33 is formed from a porous material similarly to the carrier. The absorption pad 33 absorbs the sample solution 70, the first treatment liquid 42, and the second treatment liquid 52, which are developed on the carrier 30.

The back pressure-sensitive adhesive sheet 34 is a base material of which the surface is a pressure-sensitive adhesive surface, and the carrier 30 is adhesively fixed thereto.

As shown in Fig. 5, the first container 40 accommodates the first treatment liquid 42 to be supplied to the assay region L1 in which the sample has been developed. The first container 40 is a container having an opening on one surface, and includes a film 41 that covers the opening in a liquid-tight manner. The film 41 is, for example, a sealing film such as an aluminum sheet, and can be easily broken.

The second container 50 accommodates the second treatment liquid 52 to be supplied to the assay region L1 in which the first treatment liquid 42 has been developed. The second container 50 is a container having an opening on one surface, and includes a film 51 that covers the opening in a liquid-tight manner. The film 51 is, for example, a sealing film such as an aluminum sheet, and can be easily broken.

As shown in Figs. 4 and 5, a recess part 36 is formed at one end of an inner bottom surface of the case main body 12, and the first container 40 is accommodated in the recess part 36. As shown in Fig. 5, the first container 40 is accommodated in the recess part 36 in a posture in which the film 41 is upward such that the film 41 faces the other end of the liquid feeding pad 32.

In addition, in the case 11, the second container 50 is accommodated inside the second push-down part 20 and above the assay strip 15. As shown in Fig. 5, the second container 50 accommodates the film 51 in a posture in which the film 51 is downward such that the film 51 faces the assay strip 15.

As shown in Fig. 5 as an example, a push-in protrusion member 19A is provided in the first push-down part 19. In addition, a perforating member 54 is provided below the second container 50 at a position facing the film 51 of the second container 50.

As shown in Fig. 6, the push-in protrusion member 19A abuts the other end of the liquid feeding pad 32 in a case where the first push-down part 19 is pushed down, and pushes the other end of the liquid feeding pad 32 in toward the film 41 of the first container 40. The film 41 is broken by the pushing in of the other end of the liquid feeding pad 32 by the push-in protrusion member 19A, and the other end of the liquid feeding pad 32 is dropped into the first container 40 and immersed in the first treatment liquid 42. The first treatment liquid 42 is developed from the other end of the liquid feeding pad 32 toward the carrier 30 by the capillary action.

In addition, as shown in Fig. 6, in a case where the second push-down part 20 is pushed down, the second container 50 moves downward and reaches the perforating member 54. The film 51 is pressed against the perforating member 54, and thus the film 51 is broken by the perforating member 54. Then, the second treatment liquid 52 flows out from the second container 50 and is supplied onto the assay strip 15. The second treatment liquid 52 flows through between the inner surface of the cover member 13 facing the assay strip 15 and the assay strip 15 as a flow channel, and is supplied to the assay region L1.

The schematic configuration of the cartridge 10 is as described above. Here, the configuration of the assay apparatus 100 will be described with reference to Figs. 1 and 2.

The first operation part 120 and the second operation part 122 are operation parts operated by the user. In the present example, the first operation part 120 and the second operation part 122 are subjected to push-down operation by the user. In a case where the user operates the first operation part 120 in a state where the cartridge 10 is loaded in the loading part 112, the user's operation force is applied to the first container 40 as an external force from the outside of the case 11. As a result, the first treatment liquid 42 is supplied to the assay region L1. Similarly, in a case where the user operates the second operation part 122 in a state where the cartridge 10 is loaded in the loading part 112, the user's operation force is applied to the second container 50 as an external force from the outside of the case 11. As a result, the second treatment liquid 52 is supplied to the assay region L1.

As shown in Fig. 2, the first operation part 120 has a rectangular parallelepiped shape and is fitted into a hole portion provided in the housing 110. The first operation part 120 includes a locking part (not shown) that is locked to an edge of the hole portion 114 of the housing 110, and is biased to the upper surface 110A side of the housing 110 by a spring member 121 fixed to the internal structure 115 of the housing 110. The first operation part 120 is movable to the inside of the housing 110 by being pushed down from the outside to compress the spring member 121. A first pressing protrusion part 120A extending downward is provided on the inner surface of the first operation part 120. The first pressing protrusion part 120A is positioned on the first push-down part 19 of the loaded cartridge 10.

Similarly, the second operation part 122 has a rectangular parallelepiped shape and is fitted into a hole portion 116 provided in the housing 110. The second operation part 122 includes a locking part (not shown) that is locked to an edge of the hole portion 116 of the housing 110, and is biased to the upper surface 110A side of the housing 110 by a spring member 123 fixed to the internal structure 115 of the housing 110. The second operation part 122 is movable to the inside of the housing 110 by being pushed down from the outside to compress the spring member 123. A second pressing protrusion part 122A extending downward is provided on the inner surface of the second operation part 122. The second pressing protrusion part 122A is positioned on the second push-down part 20 of the loaded cartridge 10.

In Figs. 5 and 6 in which cross-sectional views of the cartridge 10 is shown, a positional relationship between the cartridge 10 in a state of being loaded in the assay apparatus 100 and the first and second operation parts 120 and 122. Fig. 5 shows a state where the first operation part 120 and the second operation part 122 of the assay apparatus 100 are not operated, and Fig. 6 shows a state where the first operation part 120 and the second operation part 122 of the assay apparatus 100 are operated.

As shown in Fig. 6, in a case where the first operation part 120 is pushed down, the first operation part 120 is moved downward. Accordingly, the distal end of the first pressing protrusion part 120A abuts the first push-down part 19 of the cartridge 10, and the first push-down part 19 is pushed down. In a case where the first push-down part 19 is pushed down, the film 41 of the first container 40 is broken, the other end of the liquid feeding pad is immersed in the first treatment liquid 42, and the first treatment liquid 42 is supplied to the assay strip 15.

In addition, in a case where the second operation part 122 is pushed down, the second operation part 122 is moved downward. Accordingly, the distal end of the second pressing protrusion part 122A abuts the second push-down part 20 of the cartridge 10, and the second push-down part 20 is pushed down. In a case where the second push-down part 20 is pushed down, the second container 50 is moved downward, the film 51 is broken, the second treatment liquid 52 flows out from the second container 50, and the second treatment liquid 52 is supplied to the assay strip 15.

In this way, in a case where the user operates the first operation part 120 and the second operation part 122 from the outside of the housing 110 in a state where the cartridge 10 is loaded in the assay apparatus 100, the assay apparatus is configured such that the first treatment liquid 42 and the second treatment liquid 52 provided inside the cartridge 10 can be supplied to the assay strip 15. The first operation part 120 is an operation part that supplies the first treatment liquid 42 by applying an external force to the first container 40, and the second operation part 122 is an operation part that supplies the second treatment liquid 52 by applying an external force to the second container 50.

Next, other components of the assay apparatus 100 will be described with reference to Fig. 2.

The circuit board 130 is disposed on an inner surface of the upper surface 110A of the housing 110, on which the first light emitting part 124 to the third light emitting part 126 are provided, and the first light emitting part 124 to the third light emitting part 126 are connected to the circuit board 130. The battery 138 is electrically connected to the circuit board 130, and supplies electricity to the first light emitting part 124 to the third light emitting part 126, the processor 132, the internal illumination 134, and the imaging element 136 via the circuit board 130.

The processor 132 controls the first light emitting part 124 to the third light emitting part 126, the internal illumination 134, and the imaging element 136, and determines whether the sample is positive or negative.

Each of the first light emitting part 124 to the third light emitting part 126 includes a light emitting element. The light emitting element is, for example, a light emitting diode (LED), an organic electro luminescence (EL) element, or the like. As described above, for example, the first light emitting part 124 is turned on in a case of being positive, the second light emitting part 125 is turned on in a case of being negative, and the third light emitting part 126 is turned on to notify the operation timing of the second operation part 122. As described above, the first light emitting part 124 to the third light emitting part 126 constitute a notification part that transmits information to the user. In addition, the first light emitting part 124 to the third light emitting part 126 may be turned on to notify whether the cartridge 10 is normally loaded into the loading part 112, to notify that an error has occurred, or the like. The lighting state of the first light emitting part 124 to the third light emitting part 126 is controlled by the processor 132.

The internal illumination 134 illuminates an imaging region in a case where the imaging of the imaging region including the assay region L1, the control region L2, and the color development region L3 is performed by the imaging element 136. An illumination timing of the internal illumination 134 is controlled by the processor 132. It is noted that in a case where the chemiluminescence or the light based on chemiluminescence is detected as the light from the assay region L1, the imaging is performed by the imaging element 136 in a state where the internal illumination 134 is turned off.

The imaging element 136 is an aspect of a light detection part that detects light from the assay region L1 to which the sample and the treatment liquid (here, the first treatment liquid 42 and the second treatment liquid 52) are supplied. In the present example, the imaging element 136 performs imaging of an imaging region including the assay region L1, the control region L2, and the color development region L3, and outputs the imaged image to the processor 132. The imaging element is, for example, a charge coupled device (CCD) camera or a complementary metal oxide semiconductor (CMOS) camera. The imaging element 136 performs imaging of the imaging region in a case where the positive-negative determination is performed, a case where the presence or absence of the color development in the color development region L3 is confirmed, or the like. The processor 132 analyzes the image acquired from the imaging element 136 to determine whether to be positive or negative. In addition, the processor 132 analyzes the image acquired from the imaging element 136 to confirm whether the presence or absence of the color development in the color development region L3.

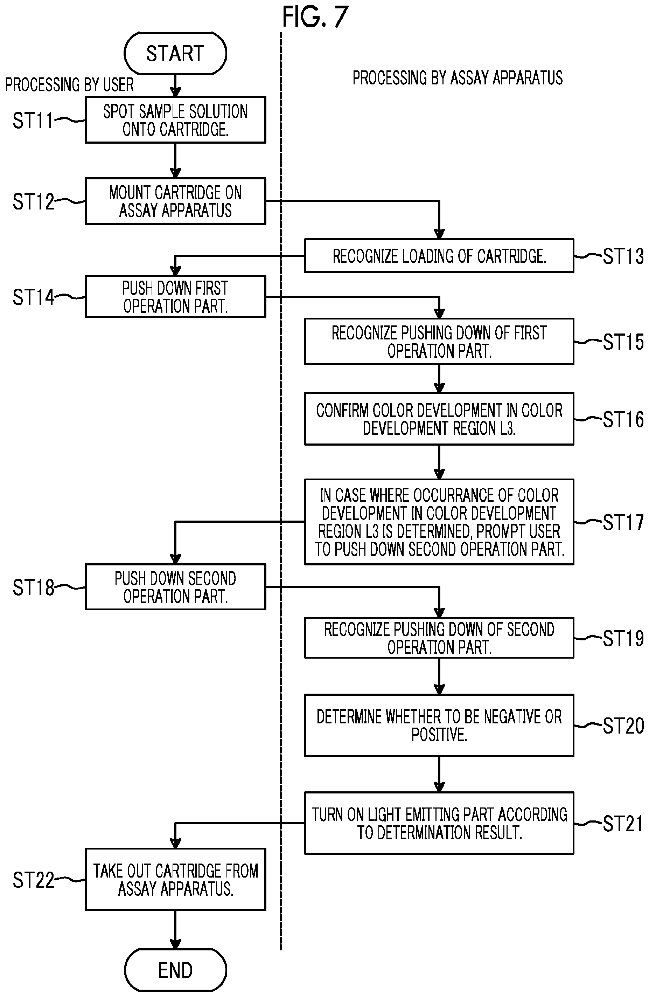

The assay procedure using the assay apparatus 100 will be described with reference to Fig. 7. In Fig. 7, the left side shows processing performed by the user, and the right side shows processing performed by the assay apparatus.

First, the sample solution is added dropwise from the sample dropping port 17 of the cartridge 10 by the user (Step ST11).

Next, the user loads the cartridge 10 into the assay apparatus 100 (Step ST12).

The assay apparatus 100 recognizes that the cartridge 10 is loaded into the loading part 112 (Step ST13). In a case where the cartridge 10 is normally loaded at the time of loading the cartridge 10, the processor 132 causes, for example, the first light emitting part 124 to blink and/or the buzzer to generate a single sound. In a case where there is an abnormality in the loading, the first light emitting part 124 to the third light emitting part 126 may be turned on and off at the same time.

Next, the user pushes down the first operation part 120 (Step ST14). In a case where the first operation part 120 of the assay apparatus 100 is pushed down by the user, the first push-down part 19 of the cartridge 10 is pushed down, and the first treatment liquid 42 is supplied to the assay strip 15. The first treatment liquid 42 is developed on the assay strip 15 and supplied to the assay region L1.

In a case where the first operation part 120 is pushed down by the user, the push-down of the first operation part 120 is recognized in the assay apparatus 100 (Step ST15).

Thereafter, in the assay apparatus 100, the confirmation processing of the color development of the color development region L3 is started with the push-down of the first operation part 120 as a trigger (Step ST16). Specifically, the processor 132 turns on the internal illumination 134, and performs imaging of a region including the color development region L3 by the imaging element 136 in a state where the color development region L3 is illuminated. The processor 132 analyzes the image acquired from the imaging element 136 and determines whether the presence or absence of the color development in the color development region L3. The processor 132 repeats the acquisition of the image by the imaging element 136 and the image analysis until the color development of the color development region L3 is confirmed or until a preset time is reached. A waiting time until the start of imaging, an imaging interval, and the like in this case are arbitrarily set. While the confirmation processing of color development is being performed, for example, the processor 132 causes the light emitting parts 124 to 126 to blink to notify the user that the processing is being performed. For example, the first light emitting part 124, the second light emitting part 125, and the third light emitting part 126 are sequentially turned on and off, or the like. In a case where the color development region L3 develops color, this indicates that the first treatment liquid 42 has reached the color development region L3 through the assay region L1 and the control region L2.

In a case where the processor 132 determines that the color development is present in the color development region L3, the processor 132 prompts the user to push down the second operation part 122 (Step ST17). That is, the user is notified of the operation timing of the second operation part 122. For example, the second operation part is prompted to be pushed down by turning on the third light emitting part 126, or the like. In addition, in a case where the color development of the color development region L3 is not recognized until the preset time is reached in Step ST16, an error is notified. For example, an error is notified by turning on and off the first light emitting part 124 to the third light emitting part 126 at the same time, or the like.

The user who has recognized the lighting of the third light emitting part 126 of the assay apparatus 100 pushes down the second operation part 122 (Step ST18). In a case where the second operation part 122 of the assay apparatus 100 is pushed down, the second push-down part 20 of the cartridge 10 is pushed down, and the second treatment liquid 52 is supplied to the assay strip 15. The second treatment liquid 52 is developed on the assay strip 15 and supplied to the assay region L1.

In a case where the second operation part 122 is pushed down by the user, the push-down of the second operation part 122 is recognized in the assay apparatus 100 (Step ST19).

Thereafter, in the assay apparatus 100, the positive-negative determination processing of determining whether to be positive or negative is started with the push-down of the second operation part 122 as a trigger (Step ST20). Specifically, the processor 132 performs imaging of a region including the assay region L1 and the control region L2 by the imaging element 136, analyzes the image acquired from the imaging element 136, and performs the positive-negative determination of the sample. The processor 132 repeats the imaging and the determination until the light emission of the control region L2 is confirmed or until a set time is reached. The waiting time until the start of imaging and the imaging interval are arbitrary. In a case where chemiluminescence is detected as the light from the assay region L1, the internal illumination is turned off during imaging. On the other hand, in a case where a change in the color development state of the assay region L1 is detected as the light from the assay region L1, the imaging is performed in a state where the internal illumination is turned on.

To notify the user that the positive-negative determination processing is being performed, the processor 132 may turn on and off the light emitting parts 124 to 126 to blink. For example, the first light emitting part 124, the second light emitting part 125, and the third light emitting part 126 are sequentially turned on and off, or the like.

The processor 132 turns on the first light emitting part 124 or the second light emitting part 125 according to the determination result (Step ST21). As an example, the processor turns on the first light emitting part 124 in a case where the determination is positive, and turns on the second light emitting part 125 in a case where the determination is negative.

The user visually recognizes the lighting states of the light emitting parts 124 to 126 and recognizes the determination result. Then, the cartridge is taken out from the assay apparatus (Step ST22), and the assay is ended.

The assay apparatus 100 includes an operation part (here, the first operation part 120 and the second operation part 122) that supplies the treatment liquid (here, the first treatment liquid 42 and the second treatment liquid 52) in the container to the assay region L1 by applying, as an external force, the operation force of the user to the container (here, the first container 40 and the second container 50) accommodated in the case 11 from the outside of the case 11 of the cartridge 10. As a result, since the actuator for supplying the treatment liquid can be omitted, the assay apparatus 100 being simple in configuration can be provided. Since the assay apparatus 100 does not include the actuator, the assay apparatus 100 can be configured to be small and low cost.

The assay apparatus 100 of the above-described embodiment is an example in which the first operation part 120 and the second operation part 122 are provided, the cartridge 10 on which the sample is spotted is mounted, and the first operation part 120 and the second operation part 122 are sequentially operated by the user. The assay apparatus for performing the assay on the cartridge 10 including the first push-down part 19 and the second push-down part 20 may include at least the second operation part 122 that is operated in a case where the second treatment liquid 52 is supplied to the assay strip 15. In such a case, the user may perform a processing of pressing the first push-down part 19 to supply the first processing liquid 42 to the assay strip 15 after spotting the sample on the cartridge 10, and then may load the cartridge 10 into the assay apparatus.

The assay apparatus 100 includes the first light emitting part 124 and the second light emitting part 125 as a notification part that notifies of whether to be negative or positive, but may be configured to include a speaker as a notification part and notify of whether to be negative or positive by voice. In addition, the assay apparatus 100 includes three light emitting parts 124 to 126, but the number of light emitting parts is not limited to three, and may be one or two. Moreover, four or more of the light emitting parts may be provided. In addition, as the notification part that notifies of the measurement state, a buzzer is provided in addition to the light emitting part. In addition to the lighting state of the light emitting part, the normal loading of the cartridge 10, the operation timing of the second operation part 122, the completion of the positive-negative determination, the occurrence of an error, and the like may be notified by a single tone of a buzzer, repetition of a single tone, a long tone, and the like.

It is noted that an example in which the battery 138 is built in the assay apparatus 100 has been described, but the assay apparatus 100 may be configured to include a power supply cord and take in electricity from the outside. Provided that since the assay apparatus 100 can be used even in an environment where there is no power supply infrastructure, it is preferable that the assay apparatus 100 includes the battery 138 inside.

In the present assay apparatus 100, the notification part constituted by the three light emitting parts 124 to 126 is provided to notify the assay result, but the assay apparatus 100 may not include such a notification part and may include a communication part to display the result on a smartphone, a tablet, or the like. In addition, the assay apparatus 100 may be configured not to include the processor 132 inside, to transmit the image imaged by the imaging element 136 to a server or the like on the cloud by the communication part, and to cause the server to have the function of the processor 132.

The configuration of the cartridge 10 is an example, and the present invention is also applicable to a cartridge having other specifications. The assay strip 15 of the cartridge 10 includes the color development region L3, and is configured such that the user can confirm the timing of the supply of the second treatment liquid 52 depending on the presence or absence of the color development in the color development region L3. However, the assay strip 15 may not include the color development region L3. In that case, the assay apparatus 100 may be configured to notify the user of the push-down timing of the second operation part 122, that is, the timing of the supply of the second treatment liquid 52 after waiting for a predetermined time after the first operation part 120 is pushed down.

The assay apparatus of the present disclosure is applicable to a cartridge in which an assay strip having an assay region L1 in which a sample is developed and a test substance contained in the sample is captured, and a container accommodating a treatment liquid to be supplied to the assay region L1 are accommodated in a case, the cartridge being capable of detecting light from the assay region from the outside. For example, the present invention can also be applied to an immunochromatographic kit (corresponding to a cartridge) disclosed in WO2017/104143A.

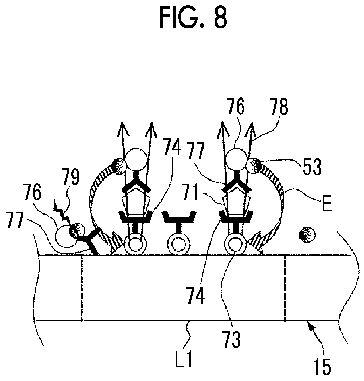

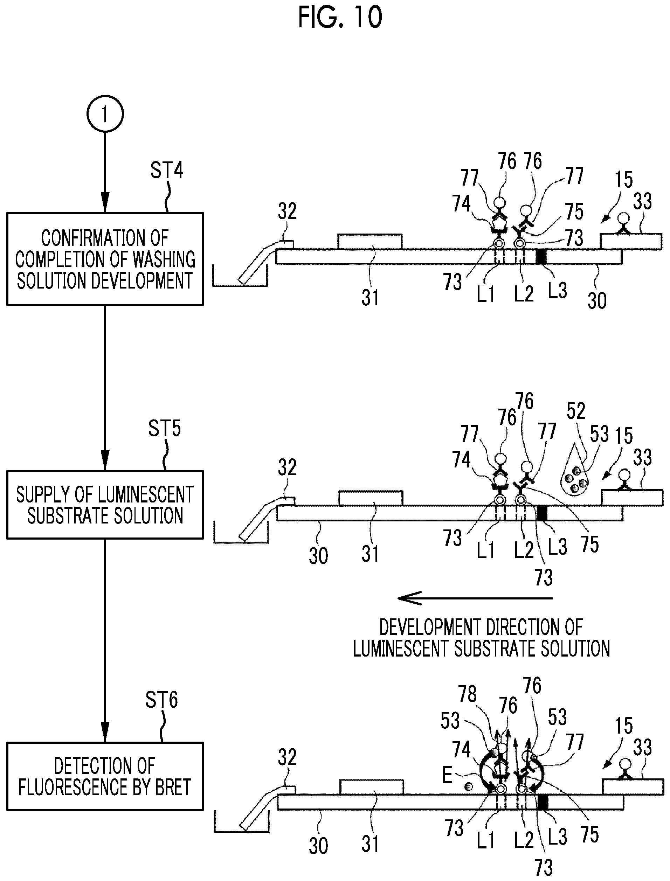

As a more specific example, a case where an assay is performed by a chemiluminescence immunochromatographic method using BRET will be described. In this case, the cartridge 10 has a configuration for performing a chemiluminescence immunochromatographic method using BRET, as the assay strip 15, the first treatment liquid 42, and the second treatment liquid 52. A specific configuration example will be described below. Fig. 8 is an explanatory view of BRET, and Figs. 9 and 10 are diagrams showing an assay procedure by a chemiluminescence immunochromatographic method using BRET.

As shown in Fig. 9, the label holding pad 31 of the assay strip 15 contains a labeled binding substance 77 labeled with the luminescent protein 76. That is, the luminescent protein 76 is used as the label.

The binding substance for capturing 74 labeled with the fluorescent substance 73 is immobilized in the assay region L1. The fluorescent substance 73 is a fluorescent dye, a fluorescent protein, or the like, which causes fluorescence to be generated in a case where excitation energy is applied.

A control binding substance 75 labeled with a fluorescent substance 73 is immobilized in the control region L2.

In this case, the first treatment liquid 42 is a washing solution (hereinafter, referred to as a washing solution 42), and the second treatment liquid 52 is a luminescent substrate solution (hereinafter, referred to as a luminescent substrate solution 52) containing a luminescent substrate 53.

The washing solution 42 is supplied to the assay region L1 after the sample is supplied to the assay region L1 and before the luminescent substrate 53 is supplied to the assay region L1. The washing solution 42 is used to wash the labeled binding substance 77 that has non-specifically adsorbed in the assay region L1. In a case where the sample solution 70 containing the sample is spotted on the label holding pad 31, the labeled binding substance 77 applied to the label holding pad 31 is developed on the carrier 30 together with the sample solution. In this case, the labeled binding substance 77 that is not bound to the test substance 71 may non-specifically adsorb to the assay region L1. Here, the "labeled binding substance 77 is non-specifically adsorbed" means that the labeled binding substance 77 is in a state of being adsorbed on the carrier 30 without depending on the specific binding between the test substance 71 and the binding substance for capturing 74. The washing solution 42 removes the labeled binding substance 77 that has non-specifically adsorbed. By removing the labeled binding substance 77 that has non-specifically adsorbed to the assay region L1 and the periphery thereof, the background during photometry can be reduced, and the S/N can be improved.

The washing solution 42 is a liquid that can wash away the labeled binding substance 77 and the test substance 71 that have non-specifically adsorbed, and does not weaken the specific binding or cause a decrease in sensitivity of light detection in later steps. Examples of the washing solution 42 include a surfactant, a buffer solution containing a preservative, and the like.

The color development region L3 contains a substance (not shown) of which the color development state changes by reacting with the washing solution 42. In a case where the color development region L3 has developed color, the color of the color development region L3 has changed, or the like, a change in the color development state of the color development region L3 means that the washing solution 42 has reached the color development region L3. The change in the color development state of the color development region L3 means that the washing solution 42 is developed up to the color development region L3 through the assay region L1 and the control region L2, and the timing at which the supply of the luminescent substrate solution 52 is started.

The luminescent substrate 53 reacts with the luminescent protein 76 and then is oxidized to generate energy E. This causes an excited state, and luminescence occurs in a case where a transition to the ground state occurs. As shown in Fig. 8, in a case where the fluorescent substance 73 and the luminescent protein 76 are positioned in a state of being close to each other, a bioluminescence resonance energy transfer (BRET) occurs in which the energy E generated by the reaction between the luminescent substrate 53 and the luminescent protein 76 is transferred to the fluorescent substance 73. As a result, fluorescence 78 is generated. That is, due to the energy E of the luminescence that occurs by the reaction between the luminescent substrate 53 and the luminescent protein 76, the fluorescent substance 73 is excited to cause fluorescence 78 to be generated from the fluorescent substance 73. In the present example, it is determined whether the sample is positive or negative by detecting the fluorescence 78 generated from the fluorescent substance 73 by this BRET. The details of the assay procedure will be described later.

Some labeled binding substances 77 may not bind to the test substance 71. Such a labeled binding substance 77 reaches the control region L2 without being captured in the assay region L1 and then is captured in the control region L2. In a case where the luminescent protein 76 is captured via the labeled binding substance 77, the fluorescent substance 73 and the luminescent protein 76 are positioned close to each other in the control region L2. In a case where the luminescent substrate 53 is developed in this state, BRET occurs in the control region L2. By detecting the fluorescence generated from the fluorescent substance 73 by BRET, it can be confirmed that the sample solution 70 is sufficiently developed on the carrier 30 and the development of the sample solution 70 is completed.

As the fluorescent substance 76 and the luminescent protein 73, those having an overlap between the wavelength range of the emission spectrum of the luminescent protein 76 and the wavelength range of the excitation spectrum of the fluorescent substance 73 are selected. Energy transfer can occur in a case where there is a wavelength range in which the emission spectrum of the luminescent protein 76 overlaps with the excitation spectrum of the fluorescent substance 73.

The luminescent protein 76 is, as an example, a luminescent protein that emits blue light. In this case, the fluorescent substance 73 can be allowed to be, as an example, a green fluorescent dye or a green fluorescent protein, which emits green fluorescence. Specific examples of the combination of the luminescent protein 76, the fluorescent substance 73, and the luminescent substrate 53 include a combination of NanoLuc (registered trade name, manufactured by Promega Corporation) which is a blue luminescent protein, mNeongreen (manufactured by Allele Biotechnology and Pharmaceuticals, Inc.) which is a green fluorescent protein, and Furimazine (manufactured by Promega Corporation).

Hereinafter, a procedure of the assay by a chemiluminescence immunochromatographic method using BRET will be described with reference to Figs. 9 and 10. Here, a description will be made using, as an example, a case where a sample contains the test substance 71, that is, a case where the sample is positive. It is noted that in Figs. 9 and 10, the back pressure-sensitive adhesive sheet 34 is not illustrated in the drawing.

First, as shown in a step ST1, the sample solution 70 is spotted on the label holding pad 31. The test substance 71 in the sample solution 70, which has been spotted on the label holding pad 31, specifically binds to the labeled binding substance 77 of the label holding pad 31.

As shown in Step ST2, the sample solution 70 permeates from the label holding pad 31 to the carrier 30 and is developed on the assay region L1 side because of the capillary action. The test substance 71 that has reached the assay region L1 is captured by the binding substance for capturing 74. As a result, the luminescent protein 76 is captured by the binding substance for capturing 74 of the assay region L1 via the test substance 71 and the labeled binding substance 77 (see Step ST3). The labeled binding substance 77 that is not captured in the assay region L1 is further developed up to the absorption pad 33. It is noted that a part of the labeled binding substances 77 may not be developed to the absorption pad 33 and may be non-specifically adsorbed on the carrier 30.

Next, as shown in a Step ST3, the washing solution 42 is supplied. The washing solution 42 is supplied to the carrier 30 via the liquid feeding pad 32 and is developed on the assay region L1 side because of the phenomenon action. By this development of the washing solution 42, the labeled binding substance 77 that has non-specifically adsorbed on the carrier 30 is washed. In this case, the labeled binding substance 77 that has non-specifically adsorbed in the assay region L1 is allowed to flow to the absorption pad 33 together with the washing solution 42.

Thereafter, as shown in Step ST4, the washing solution 42 reaches the color development region L3, and a change occurs in the color development state of the color development region L3, thereby it is confirmed that the development of the washing solution 42 is completed.

After confirming the development completion of the washing solution 42, the luminescent substrate solution 52 is supplied as shown in Step ST5. The luminescent substrate solution 52 is supplied to the carrier 30 from one end on the opposite side of the label holding pad 31 with the assay region L1 interposed therebetween, and is developed on the assay region L1 side.

As shown in Step ST6, the luminescent substrate 53 contained in the luminescent substrate solution 52 that has reached the assay region L1 reacts with the luminescent protein 76 that has been captured in the assay region L1. Then, BRET in which the energy E generated by this reaction is transferred to the fluorescent substance 73 immobilized in the assay region L1 occurs, and the fluorescence 78 is generated from the luminescent protein 76. By detecting the fluorescence 78 from the assay region L1, it is possible to determine whether the sample is positive or negative.

In a case where the sample is positive, as shown in Step ST6 of Fig. 10, the test substance 71 is captured by the binding substance for capturing 74 of the assay region L1, and the luminescent protein 76 is captured via the test substance 71. In this state, in a case where the luminescent substrate 53 is supplied to the assay region L1, the luminescent substrate 53 reacts with the luminescent protein 76 to generate luminescence energy E, and the fluorescence 78 is generated from the fluorescent substance 73 due to BRET. In this way, in a case where the sample is positive, the fluorescence 78 due to the BRET is detected as light from the assay region L1. It is noted that in this case, since the luminescent protein 76 is also captured in the control binding substance 75 of the control region L2 via the labeled binding substance 77, the fluorescence 78 due to BRET is also detected from the control region L2.

It is noted that in a case where the sample is negative, since the test substance 71 is not contained in the sample, the test substance 71 is not captured by the binding substance for capturing 74 of the assay region L1, and as a result, the luminescent protein 76 is also not captured in the assay region L1. In this state, even in a case where the luminescent substrate 53 is supplied to the assay region L1, since the luminescent protein 76 is not present in the assay region L1, light from the assay region L1 is not detected. On the other hand, even in a case where the sample is negative, the fluorescence 78 due to BRET is generated from the control region L2. Therefore, in a case where the light from the assay region L1 is not detected, it is possible to determine that the assay is completed by detecting the fluorescence 78 from the control region L2.

In the assay apparatus 100, as described above, the positive or negative can be determined by detecting the light from the assay region L1.

In a case where the labeled binding substance that has non-specifically adsorbed to the assay region L1 or the vicinity thereof is present, BRET does not occur, and the luminescent protein 76 and the luminescent substrate 53 may react with each other to generate light 79 (see Fig. 8). Such light 79 serves as a background and hinders the detection of the fluorescence 78.

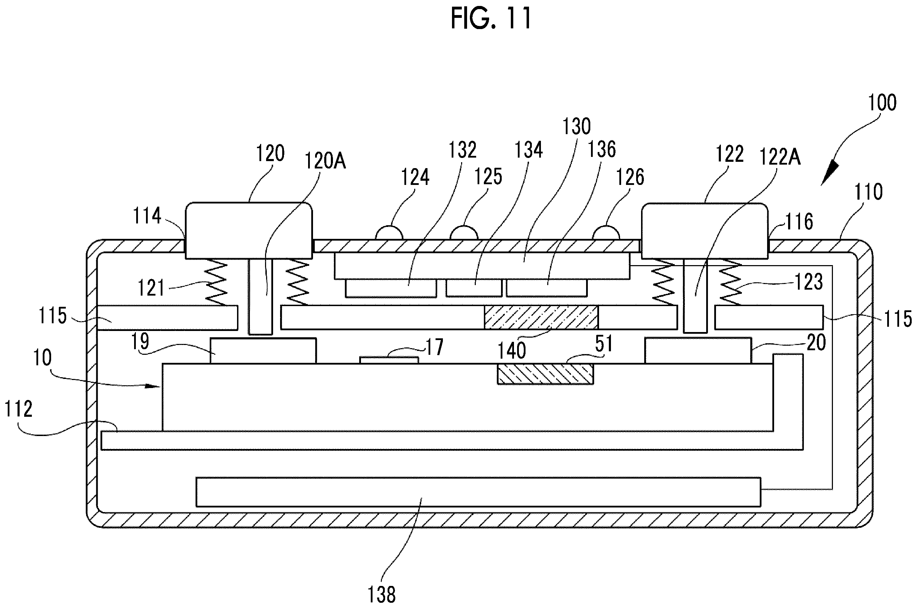

Therefore, in a case where the cartridge 10 that realizes the chemiluminescence immunochromatographic method using BRET as described above is used, as shown in Fig. 11, the assay apparatus 100 preferably includes an optical filter 140 that attenuates the light 79 generated by the reaction between the luminescent protein 76 and the luminescent substrate 53 and allows the fluorescence 78 from the fluorescent substance 73 to transmit, between the photometric window part 18 of the cartridge 10 and the imaging element 136, that is, on the optical path in which light passes from the photometric window part 18 to the imaging element 136. By providing such an optical filter 140, the light 79 incident on the imaging element 136 can be suppressed, thereby the fluorescence 78 can be detected with higher accuracy.

In the above, a case where the labeled binding substance 77 is labeled with the luminescent protein 76 and the binding substance for capturing 74 is labeled with the fluorescent substance 73 has been described as the cartridge 10 that realizes the chemiluminescence immunochromatographic method using BRET. The present invention is not limited to this aspect, and the labeled binding substance 77 may be labeled with the fluorescent substance 73 and the binding substance for capturing 74 may be labeled with the luminescent protein 76.

In addition, the assay apparatus according to the present disclosure can also be applied to a cartridge for performing a chemiluminescence immunochromatographic method without using BRET or a cartridge of an aspect in which a metal colloid is used as a label and a signal amplification treatment by silver amplification is performed.

In the former cartridge, similarly as in the case of the chemiluminescence immunochromatographic method using BRET, the first treatment liquid 42 is a washing solution, and the second treatment liquid 52 is a luminescent substrate solution. Then, as the light from the assay region L1, the light generated by the reaction between the luminescent protein and the luminescent substrate is detected.

In the latter cartridge, the first treatment liquid 42 is a first amplification liquid containing a silver ion reducing agent, and the second treatment liquid 52 is a second amplification liquid containing a silver ion. Then, reflected light by silver amplified metal colloidal label is detected as the light from the assay region L1. The reflected light is reflected light generated by turning on the internal illumination 134 and emitting the irradiation light.

In the above-described embodiments, various processors to be described below can be used as a hardware structure of a processing unit, which carries out various types of processing. For example, the various processors include, in addition to the CPU that is a general-purpose processor functioning as various processing units by executing software (operation program), a programmable logic device (PLD) such as a field programmable gate array (FPGA) that is a processor having a circuit configuration changeable after manufacture, and/or a dedicated electric circuit such as an application specific integrated circuit (ASIC) that is a processor having a circuit configuration dedicatedly designed to execute specific processing.

One processing unit may be configured by one of these various processors, or may be configured by a combination of two or more processors having the same type or different types (for example, a combination of a plurality of FPGAs and/or a combination of a CPU and an FPGA). In addition, a plurality of processing units may be configured with one processor.

As an example of configuring the plurality of processing units with one processor, first, there is a form in which one processor is configured by a combination of one or more CPUs and software and the processor functions as the plurality of processing units, as represented by computers such as a client and a server. A second example of the configuration is an aspect in which a processor that implements the functions of the entire system including a plurality of processing units using one integrated circuit (IC) chip is used. A representative example of the aspect is a system-on-chip (SoC). In this way, various processing units are configured by one or more of the above-described various processors as hardware structures.

Furthermore, more specifically, an electric circuit (circuitry) in which circuit elements such as semiconductor elements are combined can be used as the hardware structure of the various processors.

The following appendixes are further disclosed with respect to the above embodiment.

### (Appendix 1)

An immunochromatographic assay apparatus for assaying a sample, the immunochromatographic assay apparatus comprising:
a loading part that is configured to attachably and detachably load a cartridge in which an assay strip having an assay region in which the sample is developed and a test substance contained in the sample is captured, and a container that accommodates a treatment liquid to be supplied to the assay region are accommodated in a case;
an operation part that is operated by a user, in which in a state where the cartridge is loaded in the loading part, an operation force of the user is applied to the container from an outside of the case as an external force to supply the treatment liquid to the assay region; and
a light detection part that detects light from the assay region to which the sample and the treatment liquid are supplied.

### (Appendix 2)

The immunochromatographic assay apparatus according to appendix 1,
wherein the treatment liquid includes a first treatment liquid that is supplied to the assay region where the sample has been developed, and a second treatment liquid that is supplied to the assay region after the first treatment liquid is supplied,
the container includes a first container that accommodates the first treatment liquid, and a second container that accommodates the second treatment liquid, and
the operation part is an operation part that supplies the second treatment liquid to the assay region by applying an external force to the second container.

### (Appendix 3)

The immunochromatographic assay apparatus according to appendix 2, wherein the operation part includes a first operation part that supplies the first treatment liquid by applying an external force to the first container, and a second operation part that supplies the second treatment liquid by applying an external force to the second container.

### (Appendix 4)

The immunochromatographic assay apparatus according to any one of appendixes 1 to 3, further comprising: a notification part that notifies of an assay result of the sample.

### (Appendix 5)

The immunochromatographic assay apparatus according to appendix 4, wherein the notification part has a light emitting element and displays whether to be negative or positive by a lighting state of the light emitting element.

### (Appendix 6)

The immunochromatographic assay apparatus according to appendix 4, wherein the notification part notifies of whether to be negative or positive by voice.

### (Appendix 7)

The immunochromatographic assay apparatus according to any one of appendixes 1 to 6, further comprising: a processor that is configured to determine whether the sample is negative or positive based on a signal acquired from the light detection part.

### (Appendix 8)

The immunochromatographic assay apparatus according to any one of appendixes 1 to 7,
wherein the light detection part has an imaging element.

### (Appendix 9)

The immunochromatographic assay apparatus according to any one of appendixes 1 to 8,
wherein a battery that supplies electricity to the light detection part is built in.

### (Appendix 10)

The immunochromatographic assay apparatus according to appendix 4 or 5,
wherein the notification part further notifies of a timing of operating the operation part.

### (Appendix 11)

The immunochromatographic assay apparatus according to any one of appendixes 1 to 10,
wherein the light detection part detects, as the light from the assay region, light caused by a reaction of a luminescent protein which has been applied to the test substance with a luminescent substrate contained in the treatment liquid.

### (Appendix 12)

The immunochromatographic assay apparatus according to appendix 11,
wherein the light detection part detects, as the light caused by the reaction of the luminescent protein with the luminescent substrate, fluorescence generated by transferring energy of light generated by the reaction of the luminescent protein with the luminescent substrate, to a fluorescent substance which has been applied to the test substance.

### (Appendix 13)

The immunochromatographic assay apparatus according to appendix 12,
wherein the cartridge includes a photometric window part that outputs the light from the assay region to the outside, and
in a case where the cartridge is loaded into the loading part, the cartridge includes, between the photometric window part and the light detection part, an optical filter that attenuates the light generated by the reaction of the luminescent protein with the luminescent substrate, and that allows the fluorescence to be transmitted.

It is noted that the disclosure of JP2022-153101A filed on September 26, 2022, is incorporated in the present specification in its entirety by reference. All documents, patent applications, and technical standards disclosed in the present specification are incorporated in the present specification by reference to the same extent as those in a case where each of the documents, patent applications, and technical standards are specifically and individually indicated to be incorporated by reference.

## Claims

1. An immunochromatographic assay apparatus for assaying a sample, the immunochromatographic assay apparatus comprising:
a loading part that is configured to attachably and detachably load a cartridge in which an assay strip having an assay region in which the sample is developed and a test substance contained in the sample is captured, and a container that accommodates a treatment liquid to be supplied to the assay region are accommodated in a case;
an operation part that is operated by a user, in which in a state where the cartridge is loaded in the loading part, an operation force of the user is applied to the container from an outside of the case as an external force to supply the treatment liquid to the assay region; and
a light detection part that detects light from the assay region to which the sample and the treatment liquid are supplied.

2. The immunochromatographic assay apparatus according to claim 1,
wherein the treatment liquid includes a first treatment liquid that is supplied to the assay region where the sample has been developed, and a second treatment liquid that is supplied to the assay region after the first treatment liquid is supplied,
the container includes a first container that accommodates the first treatment liquid, and a second container that accommodates the second treatment liquid, and
the operation part is an operation part that supplies the second treatment liquid to the assay region by applying an external force to the second container.

3. The immunochromatographic assay apparatus according to claim 2,
wherein the operation part includes a first operation part that supplies the first treatment liquid by applying an external force to the first container, and a second operation part that supplies the second treatment liquid by applying an external force to the second container.

4. The immunochromatographic assay apparatus according to claim 1, further comprising:
a notification part that notifies of an assay result of the sample.

5. The immunochromatographic assay apparatus according to claim 4,
wherein the notification part has a light emitting element and displays whether to be negative or positive by a lighting state of the light emitting element.

6. The immunochromatographic assay apparatus according to claim 4,
wherein the notification part notifies of whether to be negative or positive by voice.

7. The immunochromatographic assay apparatus according to claim 1, further comprising:
a processor that is configured to determine whether the sample is negative or positive based on a signal acquired from the light detection part.

8. The immunochromatographic assay apparatus according to claim 1,
wherein the light detection part has an imaging element.

9. The immunochromatographic assay apparatus according to claim 1,
wherein a battery that supplies electricity to the light detection part is built in.

10. The immunochromatographic assay apparatus according to claim 4,
wherein the notification part further notifies of a timing of operating the operation part.

11. The immunochromatographic assay apparatus according to any one of claims 1 to 10,
wherein the light detection part detects, as the light from the assay region, light caused by a reaction of a luminescent protein which has been applied to the test substance with a luminescent substrate contained in the treatment liquid.

12. The immunochromatographic assay apparatus according to claim 11,
wherein the light detection part detects, as the light caused by the reaction of the luminescent protein with the luminescent substrate, fluorescence generated by transferring energy of light generated by the reaction of the luminescent protein with the luminescent substrate, to a fluorescent substance which has been applied to the test substance.

13. The immunochromatographic assay apparatus according to claim 12,
wherein the cartridge includes a photometric window part that outputs the light from the assay region to the outside, and
in a case where the cartridge is loaded into the loading part, the cartridge includes, between the photometric window part and the light detection part, an optical filter that attenuates the light generated by the reaction of the luminescent protein with the luminescent substrate, and that allows the fluorescence to be transmitted.
